(19) Europäisches Patentamt / European Patent Office / Office européen des brevets

(11) EP 1 895 862 B1

(12) **FASCICULE DE BREVET EUROPEEN**

(45) Date de publication et mention de la délivrance du brevet:
**14.09.2016 Bulletin 2016/37**

(21) Numéro de dépôt: **06777310.1**

(22) Date de dépôt: **12.06.2006**

(51) Int Cl.:
*A23L 2/70* (2006.01) *C12R 1/25* (2006.01)
*A23L 33/135* (2016.01) *A23L 2/52* (2006.01)
*A23L 2/02* (2006.01) *C12N 1/20* (2006.01)

(86) Numéro de dépôt international:
**PCT/EP2006/063109**

(87) Numéro de publication internationale:
**WO 2006/131569 (14.12.2006 Gazette 2006/50)**

(54) **PROCÉDÉ DE PRÉPARATION D'UN PRODUIT ALIMENTAIRE ENRICHI EN PROBIOTIQUE ET APPAUVRI EN ACIDES ORGANIQUES**

VERFAHREN ZUR HERSTELLUNG VON MIT PROBIOTIKA ANGEREICHERTEN NAHRUNGSMITTELN MIT GERINGEM GEHALT AN ORGANISCHEN SÄUREN

PROCESS FOR PREPARING PROBIOTIC ENRICHED AND LOW-ORGANIC ACID FOOD PRODUCTS

(84) Etats contractants désignés:
**AT BE BG CH CY CZ DE DK EE ES FI FR GB GR HU IE IS IT LI LT LU LV MC NL PL PT RO SE SI SK TR**

(30) Priorité: **10.06.2005 FR 0505911**

(43) Date de publication de la demande:
**12.03.2008 Bulletin 2008/11**

(73) Titulaire: **COMPAGNIE GERVAIS DANONE**
**75009 Paris (FR)**

(72) Inventeurs:
- **BEVERINI, Marc**
  **F-92290 Chatenay Malabry (FR)**
- **LACORRE, Christelle**
  **F-91150 Etampes (FR)**
- **FRANCOIS, Alan**
  **F-91750 Chevannes (FR)**
- **LABBE, Mickael**
  **F-91310 Montlhery (FR)**

(74) Mandataire: **Regimbeau**
**20, rue de Chazelles**
**75847 Paris Cedex 17 (FR)**

(56) Documents cités:
**EP-A- 0 113 055** **EP-A- 0 166 238**
**WO-A-00/70972** **FR-A- 2 858 630**
**US-A- 1 417 412**

- **PATENT ABSTRACTS OF JAPAN vol. 013, no. 061 (C-567), 10 février 1989 (1989-02-10) & JP 63 251070 A (KANEBO LTD), 18 octobre 1988 (1988-10-18)**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 1 895 862 B1

**Description**

La présente invention concerne un procédé de préparation d' un produit alimentaire à base de fruits, de type boisson ou purée de fruits, comprenant une concentration en probiotiques vivants, et stables préférentiellement supérieure à $10^8$ UFC/ml, une teneur forte en fruits, préférentiellement supérieure à 50 % et dont la teneur en acides organiques est diminuée de 10 à 100%, préférentiellement 30 à 70 %, encore plus préférentiellement 60% par rapport à la teneur initiale de la matrice de fruits en acides organiques.

L'ingestion de microorganismes vivants appelés probiotiques, dont certaines souches de bactéries, en particulier celles qui appartiennent au genre *Lactobacillus* sont particulièrement bénéfiques au niveau de la santé. En effet, ils ont fait l'objet de nombreuses études démontrant des effets cliniques préventifs dans des domaines variés (par exemple dans les domaines des manifestations allergiques, des diarrhées infectieuses, des maladies inflammatoires) et sur certaines fonctions physiologiques (par exemple la digestion du lactose, le transit intestinal, l'immunité). Ces probiotiques sont notamment capables de favoriser un bon fonctionnement de la flore intestinale qui est susceptible d'intéresser la population générale. En effet, ces bactéries produisent entre autre des bactériocines et de l'acide lactique qui augmentent indirectement la digestibilité des aliments, favorisent le péristaltisme intestinal, et accélèrent l'évacuation des selles. De plus, ces bactéries produisent certaines vitamines du complexe B, et favorisent en général l'absorption des vitamines et minéraux, diminuent le cholestérol sanguin, renforcent le système immunitaire et tapissent les muqueuses intestinales afin de protéger contre l'invasion et les activités des microorganismes nuisibles.

De ce fait, depuis plusieurs années, les industries agroalimentaires tentent d'incorporer de telles bactéries dans leurs produits.

De tels produits additionnés de bactéries sont traditionnellement des produits laitiers mais d'autres produits alimentaires, notamment à base de fruits, sont intéressants à développer pour le marché de l'industrie agroalimentaire.

Des produits alimentaires à base de fruits et additionnés de bactéries du genre *Lactobacillus* sont déjà connus dans l'état de la technique, par exemple dans la demande de brevet internationale WO 00/70972, et la demande de brevet européen EP 0113055.

Cependant, dans des produits alimentaires à base de fruits additionnés de lactobacilles, on a pu observer une croissance bactérienne qui induit lors du stockage des produits, une altération de leurs qualités par une production de gaz et de faux-goûts qui les rend impropres à la consommation.

De nombreux micro-organismes sont capables de décarboxyler des acides cinnamiques substitués tel que l'acide trans-4-hydroxy-3-methoxy-cinnamique (acide férulique) et l'acide trans-4-hydroxy-cinnamique (acide p-coumarique) pour former respectivement les deux composés volatils suivant : le 3-methoxy-4-hydroxystyrene (4-vinyl guaiacol) et le 4-hydroxystyrene (4-vinyl phénol). Ces molécules sont responsables de faux-goûts de type «phénolique, fumé, gants, médicinal... ». Les activités acide p-coumarique et acide férulique décarboxylases ont été détectées chez les bactéries du genre *Lactobacillus.* En particulier, les Lactobacilles connus pour ces activités sont les suivants : L. brevis, L. crispatus, L. fermentum, L. plantarum, L. pentosus, L. paracasei (réf biblio : van Beek, S and Priest FG - 2000 - Decarboxylation of subsituted cinnamic acids by lactic acid bacteria isolated during malt whisky fermentation - Applied and Environmental Microbiology, 66 (12) : 5322-8). Ainsi, via des voies de biotransformation, des souches de lactobacilles sont capables de générer des faux-goûts à partir d'acides phénoliques.

Actuellement, les solutions proposées dans l'état de la technique pour résoudre ce problème de production de gaz et de faux-goûts sont, par exemple dans la demande de brevet internationale WO 00/70972 déposée par la société PROBI, une conservation des produits à une température comprise entre 4 et 8°C et une faible concentration en fruits (25% environ).

Cependant ces solutions ne permettent pas de proposer des produits ayant une concentration supérieure à 50 % en fruits, comprenant en outre des bactéries du genre *Lactobacillus* vivantes et stables à une concentration importante. Ce que l'on entend par concentration importante est une population supérieure ou égale à $10^8$ UFC/ml de produit. Par bactéries stables, on entend une population bactérienne à activité métabolique réduite (production de gaz, et/ou de faux-goûts, et acidification lors du stockage limitées et maîtrisées) à froid, c'est-à-dire à une température comprise entre 4 et 10°C. La postacidification (acidification spécifique de la conservation du produit) limitée sera la conséquence d'une part d'une diminution de la concentration des acides organiques présents dans le produit et d'autre part de la basse température de conservation dudit produit.

Un produit alimentaire à base de fruits de type boisson ou purée de fruits et comprenant des probiotiques vivants stables aura comme avantage d'apporter au consommateur les bienfaits des fruits et des probiotiques.

Le Plan National Nutrition Santé préconise la consommation minimum de cinq portions de fruits et légumes par jour. Les observations menées par de nombreux scientifiques montrent que consommer plus de fruits et légumes permet notamment de réduire le taux de cholestérol, les apports lipidiques, et de limiter la prévalence de l'obésité chez les enfants.

Plusieurs études scientifiques suggèrent que les probiotiques peuvent jouer également un rôle de premier plan sur la santé. Chaque souche de probiotique peut offrir des bénéfices santé spécifiques. On peut retrouver parmi ces

bénéfices santé : une amélioration du fonctionnement du système digestif et un renforcement des défenses naturelles. Certains probiotiques agissent en absorbant des protéines et d'autres produisent des vitamines. Certains peuvent également produire des composés qui luttent contre la propagation de bactéries pathogènes et peuvent ainsi jouer un rôle dans l'écosystème intestinal.

Il serait souhaitable pour l'industrie agroalimentaire de pouvoir préparer de tels produits alimentaires, et c'est l'objet de la présente invention.

Afin d'augmenter la viabilité des bactéries les demandes de brevet européen EP 0113055 et EP 0166238 déposées par Kirin Beer proposent la réduction sélective de la concentration en polyphénols présents dans les jus de fruits, qui sont des composants bactériostatiques, par contact du jus avec des agents absorbants. Dans ce cas, le but recherché est également de favoriser la fermentation des bactéries et non de maintenir la population initiale stable comme selon la présente invention.

Dans l'état de la technique, il est connu que les fruits contiennent des acides organiques, et les inventeurs ont observé que les bactéries du genre *Lactobacillus* métabolisent ces acides organiques, et que cette métabolisation des acides organiques peut être responsable de la production du gaz carbonique, et/ou des faux-goûts dans les produits alimentaires à base de fruits. Les compositions en acides organiques de certains fruits peuvent être facilement connues en consultant des sources bibliographiques comme par exemple les échelles d'acidité par fruits dans le « code of practice » de l'AIJN.

De nombreuses souches des genres *Leuconostoc, Streptococcus* et *Lactobacillus* sont capables de dégrader le malate, le citrate, le pyruvate, le fumarate, le tartrate et le gluconate pour produire du gaz. En comparaison avec la mesure de gaz produit, la détermination des teneurs en diacétyle et en acétoïne est une méthode plus efficace pour détecter la dégradation du pyruvate (Hegazi F.Z., Abo-Elnaga I.G., 1980. Dégradation of organic acids by dairy lactic acid bacteria. Mikrobiologie der Landwirtschaft der Technologie und des Umweltschutzes, 135 (3), 212.).

Des acides organiques tels que l'acide malique ou l'acide citrique, quand ils sont dégradés, à moins que cette assimilation ne s'accompagne d'une trop grande production d'acétate qui génère lui aussi des faux-goûts, ne posent pas cette problématique justement de génération de goûts désagréables pour le consommateur. Néanmoins, l'assimilation de ces acides organiques par des souches bactériennes produira cette fois du $CO_2$ qui fera gonfler l'emballage du produit. En effet, ces acides organiques sont naturellement métabolisés par certaines espèces de lactobacilles pour produire du pyruvate (le composé central des cycles métaboliques tel que le métabolisme carboné) et du $CO_2$ ; de plus le pyruvate est lui-même sujet à des décarboxylations augmentant d'autant le taux de $CO_2$ produit.

Certains de ces acides organiques sont des composés phénoliques (acide coumarique, acide ferrulique) et la dégradation de ces composés par les souches bactériennes pourra générer des faux-goûts dans le produit.

Selon le pH du produit fini, le profil sensoriel d'un acide est très variable Ainsi, l'acide lactique est le plus astringent à pH 3,5 devant l'acide citrique et l'acide malique (Hartwig P., McDaniel M.R., 1995. Flavor characterisitics of lactic, malic, citric and acetic acids at various pH levels. Journal of Food Science, 60 (2), 384-388.).

La figure 1 représente ainsi le mécanisme de métabolisation de l'acide malique (ou malate), de l'acide citrique (ou citrate) et du pyruvate.

Il existe des boissons commerciales, notamment le produit ProViva distribué par Skanemejerier, contenant une souche du genre *Lactobacillus* à une teneur au maximum égale à $10^8$ UFC/mL et à pH acide (pH=3,8). La stabilité du produit n'est cependant garantie que grâce à une conservation à 4°C et à une faible teneur en jus de fruits (<25%). De plus, les produits commerciaux ProViva ont ciblé certains types de fruits à teneurs faibles en acides organiques et ne comprennent pas des jus importants comme les jus d'orange, les jus de pomme et les jus multifruits exotiques.

Les inventeurs ont montré qu'un appauvrissement en acides organiques de la matrice de fruits à la base du produit alimentaire permet de réduire ou d'éliminer la production du gaz carbonique, et/ou des faux-goûts dans le produit alimentaire final, après conditionnement tout en préservant les qualités nutritionnelles du produit, quelque soit les fruits, la concentration en fruits et la concentration en acides organiques du produit final. Ainsi, les inventeurs proposent de contrôler et de recommander une teneur limitante en acides organiques qui doit être ajustée à la teneur cible en matrice de fruits dans la formule et au type de fruit considéré.

Un objet de la présente invention est donc un procédé de préparation d' un produit alimentaire conditionné à base de fruits comprenant des probiotiques vivants stables et dont la teneur en acides organiques est diminuée de 10 à 100%, préférentiellement 30 à 70 %, encore plus préférentiellement 60% par rapport à la teneur initiale de la matrice de fruits en acides organiques et dans lequel la production de faux goûts est réduite ou éliminée par rapport à la matrice de fruits initiale.

Par probiotique, on entend désigner des microorganismes vivants qui, lorsqu'ils sont intégrés en quantité suffisante, exercent un effet positif sur la santé au-delà des effets nutritionnels traditionnels.

Par probiotiques vivants, on entend désigner, selon la présente invention, des probiotiques dont le taux de survie après 28 jours dans un produit alimentaire selon la présente invention est supérieur à 60% et avantageusement supérieur à 80%.

La viabilité des probiotiques est mesurée par des techniques de numération connue de l'homme du métier

comme par exemple la numération en masse, la numération en surface, les cellules de Malassez, le comptage direct, la turbidité, la néphélométrie, le comptage électronique, la cytométrie en flux, la fluorescence, l'impédancemétrie, l'analyse d'images.

Par probiotiques stables, on entend désigner, selon la présente invention, des probiotiques ayant une absence d'activité pendant au moins 30 jours à 10°C. L'absence d'activité se traduit par :

- Une absence de détection de production de gaz ($CO_2$ par exemple) lors de la conservation en emballage
- Une qualité organoleptique constante sans altération des qualités initiales de la matrice de fruits et sans production de faux goût.
- Une absence de post-acidification importante (baisse de pH< 0,5 unité).
- Une non-prolifération des probiotiques et un maintien de la population initiale (+/- 50%).

Les souches bactériennes selon la présente invention sont des souches de *Lactobacillus plantarum* déposées le 4/04/02 sous le numéro CNCM I-2845 à la Collection Nationale des Cultures de Microorganismes.

La souche *Lactobacillus Plantarum* déposée le 16/03/95 sous le numéro DSM 9843 à la Deutsche Sammlung von Mikroorganismen von Zellkuturen GmbH est commercialisée par la société PROBI, sous le nom *Lactobacillus plantarum* 299v®. Cette souche possède de nombreux avantages pour une utilisation en tant que probiotique dans un produit alimentaire à base de fruits :

- Elle répond aux critères probiotiques fixés par la communauté scientifique.
- Elle est brevetée, caractérisée (RAPD, ribotypage) et sa classification est confirmée ;
- Elle est GRAS (de l'anglais "Generally Recognized As Safe") ;
- Elle est déjà présente à un taux de $10^8$ UFC/ml dans le produit ProViva® distribué par Skanemejerier et est consommée depuis 1994 ;
- Elle présente une très bonne survie à pH acide inférieur à 4;
- Elle est amylase négative donc ne dégrade pas la texture du produit fini.

Cependant, cette souche a aussi plusieurs inconvénients :

- Elle présente un fort potentiel de post acidification
- Elle engendre des défauts organoleptiques importants liés à la synthèse d'acide acétique.
- Elle dégrade l'acide citrique (e.g jus de citron, jus d'orange) ou l'acide malique (e.g jus de pomme ou de poire) avec production de gaz carbonique d'où des problèmes de gonflement possibles notamment si la chaîne du froid est rompue (c'est à dire dépassement de la température de 8°C).

Cette souche présente donc de nombreux points positifs mais son utilisation dans le cadre de produits alimentaires à base de fruits ne peut pas se faire telle quelle, sans appauvrissement en acides organiques de la matrice de fruits à la base du produit alimentaire.

Il en est de même pour la souche de *Lactobacillus plantarum* déposée le 4/04/02 sous le numéro CNCM I-2845 à la Collection Nationale des Cultures de Microorganismes.

Par matrice de fruits, on entend désigner selon la présente invention un jus de fruits, un jus de fruits reconstitué à base de concentré, ou une purée de fruits, sans probiotiques, non appauvrie en acides organiques, mais comprenant de manière optionnelle d'autres substances telles que par exemple, du sucre, de l'eau, des arômes, des colorants, des édulcorants, des agents anti-oxygène, du lait, des conservateurs, des acidifiants, des agents de texture, des protéines d'origine animale (protéines de lait, de lactosérum...) ou végétale (soja, riz...) ou des extraits de végétaux (soja, riz...).

Par faux goûts, on entend un goût anormal pour le produit alimentaire. Un faux-goût est désagréable pour le consommateur donc non-recherché. Ainsi, à titre d'exemple on peut citer pour les produit alimentaire selon la présente invention le faux-goût de type « terreux-foin » via la fermentation et l'oxydation du produit, de type « vinaigre » via le ferment à partir d'acides organiques présent dans le produit, et de type « rance » via la présence d'acides gras volatiles.

Des notes dites « positives » peuvent également être détectées dans le produit, telles que par exemple des notes de type « orange » ou « fruitée ». Ces goûts n'étant pas désagréables pour le consommateur, ils ne sont pas compris dans les « faux-goûts » selon la présente invention.

La teneur en molécules responsables de "faux-goûts" est mesurée par micro-extraction en phase solide (SPME) associée à un chromatographe en phase gazeuse (CPG)couplé à un spectromètre de masse (SM). Cette méthode a été développée spécifiquement et possède une sensibilité accrue tout en ayant une bonne reproductibilité et une bonne répétabilité. La SPME permet une concentration spécifique des molécules volatiles cibles pour une meilleure quantification et une meilleure identification. La CPG permet la séparation des molécules volatiles selon leur polarité et leur masse molaire et ainsi obtenir des pics correspondants à chaque molécule. La teneur de chaque molécule est exprimée

en aire de pic c'est à dire en unité d'absorbance (UA) proportionnelle à leur concentration dans l'échantillon. Enfin, le spectromètre de masse permet d'une part une identification certaine de chaque molécule via leur fragmentation en ions caractéristiques et d'autre part une deuxième quantification des molécules volatiles où la teneur est cette fois exprimée en unité de masse.

Par arômes on entend désigner des ingrédients destinés à donner une flaveur (c'est à dire un goût et/ou une odeur) à une denrée alimentaire.

Les arômes sont utilisés dans deux buts technologiques principaux :

- soit ils renforcent la flaveur naturelle du produit alimentaire, ou la restituent partiellement si elle est trop faible (produits ayant perdu une partie de leur goût au cours du procédé de fabrication),
- soit ils remplacent un ingrédient apportant une note aromatique au produit fini (ex : yaourt arôme fraise).

Selon la présente invention, les arômes préférés sont : Pomme, orange, fruits rouges, fraise, pêche, abricot, prune, framboise, mure, groseille, citron, agrume, pamplemousse, banane, ananas, kiwi, poire, cerise, noix de coco, fruits de la passion, mangue, figue, rhubarbe, melon, multifruits, fruits exotiques, vanille, chocolat, café, cappuccino.

Par colorants on entend des substances capables de rendre au produit alimentaire, de renforcer ou de conférer une coloration.

Selon la présente invention, les colorants préférés sont : le bêta carotène, le carmin.

Par édulcorant, on entend des substances capables de mimer le pouvoir sucrant du sucre sans pour autant apporter les calories du sucre.

Selon la présente invention, les édulcorants préférés sont : aspartame, acésulfame K, saccharine, sucralose et cyclamate.

Par agents anti-oxygène, on entend des substances capables d'éviter ou de réduire les phénomènes d'oxydation qui provoquent entre autres le rancissement des matières grasses ou le brunissement des fruits et légumes coupés.

Selon la présente invention, les agents anti-oxygène préférés sont : vitamine C, vitamine E, extrait de romarin.

Par lait on entend le lait d'origine animale (par exemple vache, chèvre, brebis) ou des jus d'origine végétale (par exemple jus extrait de soja, tonyu, riz, avoine, quinoa, châtaigne, amande ou noisette).

Par conservateurs on entend des substances destinées à aider à la conservation en empêchant la présence et le développement de microorganismes indésirables (par exemple : moisissures ou bactéries responsables d'intoxications alimentaires) dans le produit alimentaire final.

Selon la présente invention, les conservateurs préférés sont l'acide sorbique, l'acide ascorbique, et l'anhydride sulfureux.

Par agents de texture on entend des substances qui permettent d'améliorer la présentation ou la tenue du produit alimentaire final. Les agents de texture peuvent être des émulsifiants, des stabilisants, des épaississants, ou des gélifiants. Ils peuvent être utilisés dans le produit alimentaire selon la présente invention seuls ou en combinaison.

Selon la présente invention, les agents de texture préférés sont : la pectine, utilisé comme gélifiant, la graine de caroube, les carraghénanes, les alginates, la gomme guar, la gomme de xanthane, l'amidon, les mono et diglycérides d'acides gras alimentaires.

Par acidifiants on entend préférentiellement l'acide lactique et/ou l'acide citrique et/ou l'acide ortho-phosphorique.

Par eau, on entend de manière optionnelle l'eau osmosée. L'eau osmosée permet de limiter la quantité de minéraux présents dans le produit final, les minéraux pouvant être responsables également de faux-goûts.

Le potassium, le chlore, le magnésium et le calcium sont en effet plutôt amers sous différentes formes (KCl, $NH_4CL$, $CaCl_2$, acétate de Ca, LiCl, $MgSO_4$...) alors que le sodium, le lithium et le sulfate sont plutôt salé et/ou acide selon la forme sous laquelle ils sont (forme salée : NaCl, $Na_2SO_4$, tartrate de Na ; forme acide : $Na_2NO_3$, acétate de Li ; forme salée et acide : acétate de Na, ascorbate de Na, citrate de Na). Outre ces effets directs sur les qualités sensorielles des produits, ces composés peuvent également avoir un effet « salting out » sur les molécules volatiles responsables de faux goûts de type « fumé, phénolique... » en favorisant leur passage dans la phase vapeur au dessus du produit, augmentant ainsi l'intensité des faux-goûts perçues.

Par acides organiques, on entend désigner, selon la présente invention, notamment l'acide malique, l'acide citrique, l'acide tartrique, l'acide pyruvique, l'acide fumarique, ou l'acide gluconique.

De manière préférentielle, les acides organiques dont la teneur est diminuée ou éliminée par rapport à la teneur initiale de la matrice de fruits sont les acides malique et/ou citrique.

La teneur initiale de la matrice de fruits en acides organiques peut être connue par des sources bibliographiques. Dans le cas où la matrice de fruits est un jus de fruit ou un jus de fruits reconstitué à base de concentré, les sources bibliographiques portent sur la concentration en acides organique des jus de fruits. De telles sources sont par exemple les tableaux extrait du code of practice on absolute quality requirement for juices de l'AIJN tel que celui présenté ci après :

**Tableau 1** : extrait du code of practice on absolute quality requirement for juices de l'AIJN présentant la concentration en acides organique des jus de fruits

| | Acide citrique (g/L) | Acide L-malique (g/L) |
|---|---|---|
| orange | 6.7-17 | 0.8-3 |
| Pamplemousse | 8-20 | 0.2-1.2 |
| Pomme | 50-200 | min. 3 |
| raisin | Max 0.5 | 2.5-7 |
| Ananas | 3-11 | 1.0-4.0 |
| Citron | 45-63 | 1.0-7.5 |
| Fruit de la passion | 25-50 | 1.3-5.0 |
| Poire | max. 4 | 0.8-5 |
| abricot | 1.5-16 | 5-20 |
| cassis | 26-42 | 1-4 |
| griotte | Max 0.4 | 15.5-27 |
| framboise | 9-22 | 0.2-1.2 |
| fraise | 5-11 | 0.6-5 |
| pêche | 1.5-5 | 2-6 |
| mandarine | 6-22 | 0.5-3 |

Dans le cas où la matrice de fruit n'est pas un jus de fruit ou un jus de fruits reconstitué à base de concentré, les sources bibliographiques portent sur la concentration en acides organique des fruits. De telles sources sont par « La composition des aliments : tableaux des valeurs nutritives. 2000 (6ème Edition); SOUCI S.W.; FACHMANN W.; KRAUT H.; SCHERZ H. » dont un exemple de tableau est reproduit ci après :

**Tableau 2** : concentration en acides organique des fruits

| fruit | acide citrique (mg/100g) | | | acide malique (mg/100g) | | |
|---|---|---|---|---|---|---|
| | moyenne | mini | max | moyenne | mini | max |
| poire | 140 | 80 | 200 | 170 | 100 | 240 |
| fraise | 748 | 670 | 940 | 303 | 90 | 340 |
| pêche | 240 | 160 | 320 | 330 | 280 | 370 |
| ananas | 630 | 580 | 670 | 94 | 87 | 100 |
| raisin | 23 | | | 327 | 220 | 650 |
| pomme | 29 | 9 | 30 | 426 | 270 | 790 |
| abricot | 400 | 140 | 700 | 1000 | 700 | 1300 |
| orange | 1042 | 600 | 1880 | 89 | 40 | 190 |
| banane | 201 | 80 | 390 | 360 | 240 | 500 |
| mangue | 264 | 200 | 327 | 74 | | |
| griotte | 4,7 | | | 1800 | | |
| cerise (sweet) | 13 | 10 | 15 | 940 | 730 | 1110 |
| prune | 34 | 23 | 55 | 1220 | 820 | 1990 |
| pruneau | 158 | | | 5690 | | |
| mûre | 18 | 15 | 21 | 900 | 860 | 950 |
| myrtille | 523 | | | 850 | | |
| framboise | 1720 | 1060 | 2480 | 400 | 0 | 800 |
| pamplemousse | 1296 | 1000 | 1460 | 180 | 50 | 310 |
| goyave | 537 | 532 | 541 | 325 | 182 | 469 |
| kiwi | 995 | 980 | 1010 | 500 | 470 | 530 |
| passion | 3250 | | | 650 | | |
| papaye | 54 | 29 | 100 | 29 | 27 | 31 |
| citron | 4683 | 3500 | 7200 | 200 | | |
| coing | | | | 930 | | |
| baie d'églantier | | | | 3100 | | |
| litchi | 16 | | | 239 | | |
| grenade | 500 | | | 100 | | |
| melon | 75 | 0 | 150 | - | 0 | 50 |

Dans les deux cas, la valeur du tableau utilisée pour déterminer la teneur initiale du jus de fruit ou des fruits en acides organiques est la valeur minimale.

En outre, la teneur initiale de la matrice de fruits en acides organiques peut être définie par tout méthode de quantification appropriée.

De telles méthodes sont par exemple

- la mesure de l'acidité titrable, qui quantifie la teneur en acides présents dans la matrice de fruits. Elle consiste à neutraliser un échantillon de la matrice de fruits par une solution de soude à 0,1 N, la quantité de soude nécessaire pour atteindre un pH 8 permettant de déduire la valeur d'acidité totale.
- Le dosage chromatographique par l'HPAEC (High Performance Anion Exchange Chromatography (méthode Dionex : 164-166 avenue Joseph Kessel 78960 Voisins Le Bretonneux France) couplée à une détection conductimétrique.
- les acides malique et citrique peuvent être dosés par méthodes enzymatiques ; les méthodes de référence sont préconisées par l'International Fédération of Fruit Juice Producers (IFU) (ces références sont établies depuis 1985) : IFU 21 pour l'acide malique et IFU22 pour l'acide citrique ; Il s'agit de méthodes spectrophotométriques qui font intervenir des réactions enzymatiques.

La méthode IFU 21 fonctionne sur le principe suivant : l'acide citrique (citrate) présent dans la matrice de fruits initialement est converti en oxaloacetate et acétate dans la réaction catalysée par l'enzyme citrate lyase (CL) (1).

$$\text{(1) Citrate} \xrightarrow{\text{CL}} \text{oxaloacetate + acetate}$$

En présence des enzymes L-malate dehydrogenase et L-lactate dehydrogenase, l'oxaloacétate et le pyruvate produit par décarboxylation sont réduit en L-malate et L-lactate, respectivement, par Nicotinamide Adénine Dinucléotide (NADH) (2) (3)

$$\text{(2) Oxaloacetate + NADH + } H^+ \xrightarrow{\text{L-MDH}} \text{L-Malate + } NAD^+$$

$$\text{(3) Pyruvate + NADH + } H^+ \xrightarrow{\text{L-LDH}} \text{L-Lacate + } NAD^+$$

La quantité de NADH oxydé dans les réactions (2) et (3) est stoechiométrique avec la quantité de citrate. NADH est déterminé par la mesure de son absorbance de la lumière à 334, 340 ou 365 nm. Cette mesure permet de déterminer la quantité d'acide citrique présent initialement dans la matrice de fruits.

La méthode IFU 22 fonctionne sur le principe suivant : l'acide L-lactique (L-lactate) présent dans la matrice de fruits initialement est oxydé en pyruvate par Nicotinamide Adénine Dinucléotide (NAD) en présence de L-lactate dehydrogenase (L-LDH) (1).

$$\text{(1) L-Lactate + NAD}^{+} \xrightleftharpoons{\text{L-LDH}} \text{pyruvate + } NADH^+ + H^+$$

L'équilibre de cette réaction repose du côté du L-Lactate. En piégeant le pyruvate dans une réaction ultérieure catalysée par l'enzyme glutamate-pyruvate-transaminase (GPT) en présence de L-glutamate, l'équilibre peut être déplacé en faveur du pyruvate et de NADH (2).

$$\text{(2) Pyruvate + L-glutamate} \xrightleftharpoons{\text{GTP}} \text{L-alanine + 2-oxoglutarate}$$

La quantité de NADH formé dans la réaction précédente est stoechiométrique avec la quantité d'acide L-lactique. L'augmentation de NADH est déterminée par la mesure de son absorbance de la lumière à 334, 340 ou 365 nm. Cette mesure permet de déterminer la quantité d'acide L-lactique présent initialement dans la matrice de fruits.

Selon un premier aspect de présente invention, le produit alimentaire peut être une boisson, préférentiellement à base de jus de fruits ou de jus de fruits reconstitués à base de concentré.

Selon la présente invention, on peut citer comme jus de fruits des jus d'orange et notamment le NFC (de l'anglais « Not From Concentrate ») 10-12° Brix et comme jus d'orange reconstitué à base de concentré le FCOJ (de l'anglais «Frozen Concentrate Orange Juice ») à 66° Brix et les autres jus de fruits concentrés entre 10 et 70° Brix.

Le produit alimentaire préparé selon le procédé de l'invention comprend entre 50 et 99,99de jus ou de purée de fruits.

Les probiotiques sont à une concentration comprise entre $5.10^5$ et $1.10^9$ UFC/ml, et préférentiellement à une concentration supérieure ou égale à $10^8$ UFC/ml. De manière préférée entre toute la concentration est de $4.10^7$ UFC/ml

Le produit alimentaire a un pH compris entre 3 et 4.

Le produit alimentaire se conserve, et peut donc être consommé, pendant au moins 30 jours à une température d'au plus 10°C, sans nécessiter l'adjonction d'agents bactériostatiques.

Le produit alimentaire est à base d'un fruit.

Le produit alimentaire est à base de plusieurs fruits.

Le ou les fruit(s) sont riches en acides organiques.

Les fruits sont : orange, citron, raisin, ananas, pomme, poire, pêche et/ou fruits rouges.

De manière préférentielle, le produit alimentaire comprend selon du lait et/ou jus végétal.

De manière préférentielle, le jus végétal sera un jus réalisé à partir de graines de soja (jus extrait de soja et/ou tonyu).

Les acides organiques préférentiellement éliminés de la matrice de fruits sont l'acide malique, l'acide citrique, l'acide tartrique, l'acide pyruvique, l'acide fumarique, l'acide gluconique, l'acide p. coumarique et/ou l'acide caféique.

Un objet de la présente invention est un procédé de préparation d'un produit alimentaire ayant les caractéristiques de la revendication 1.

Selon la présente invention, l'étape a) d'appauvrissement en acides organiques d'une matrice à base de fruits est réalisée par sélection d'une matrice de fruits à faible acidité naturelle.

Par matrice de fruits à faible acidité naturelle on entend désigner selon la présente invention une matrice de fruits à partir de laquelle on obtient un jus naturellement faiblement acide qui a son acidité comprise entre la valeur basse indiquée sur le « Code of Practice » de l'AIJN, (The Association of the Industry of Juice and Nectars from Fruits and Vegetables of the European Union) reconnue par tous les professionnels dans le domaine des jus de fruits et -50% de cette valeur.

Les acidités naturelles dépendent non seulement du fruit, mais aussi de sa variété, du climat, du moment de la récolte. De ce fait, des échelles d'acidité par fruit sont définies et les valeurs sont présentées dans le tableau ci-dessous : (source : AIJN):

**Tableau 3 :** échelle d'acidité des fruits selon l'AIJN

| Fruits | Acidité titrable (exprimée en g d'acide citrique anhydre par litre de jus, mesuré à pH 8.1) |
|---|---|
| Orange | 5-15 |
| Pamplemousse | 7,7-18,5 |
| Pomme | 2,2-7,5 |
| Raisin | 4-11 |
| Ananas | 3,2-11,5 |
| Citron | 44,8-62 |
| Fruit de la Passion | 25,6-50 |
| Poire | 1,4-7 g/kg |
| Abricot | 6,4-19,2 g/kg |
| Cassis | 26,7-40,1 |
| Cerise aigre | 12,8-22,6 |
| Framboise | 12,2-20 |
| Fraise | 5,1-11,5 |
| Pêche | 3,2-8 g/kg |
| Banane | 2-3,8 |
| Mandarine | 5,8-19,2 |

Pour l'orange, les variétés trouvées en faible acidité naturelle peuvent être à 3 en acidité par exemple (soit 40% en dessous de la valeur basse de l'échelle).

Selon la présente invention la sélection d'une matrice de fruits à faible acidité naturelle est réalisée par sélection variétale des fruits et/ou contrôle de la maturité des fruits.

Les fruits seront sélectionnés de manière préférentielle dès qu'ils auront atteint une maturité tardive.

Selon la présente invention l'étape a) d'appauvrissement en acides organiques d'une matrice à base de fruits est réalisée par déacidification de la matrice de fruits.

Selon la présente invention la déacidification (diminution de l'acidité titrable) de la matrice de fruits est réalisée par électrodialyse de la matrice de fruits, précipitation des acides organiques de la matrice de fruits par des sels de calcium, fermentation malolactique, assimilation sélective de l'acide citrique, et/ou passage de la matrice de fruits sur une résine échangeuse d'anions.

La fermentation citrique aboutit à la production de diacétyl-acetoine par les bactéries lactiques

De manière préférée, la désacidification de la matrice de fruits selon la présente invention est réalisée par électrodialyse et/ou passage sur résine échangeuse d'anions.

En effet, une résine échangeuse d'anions est idéale pour capter des composés qui ont des radicaux acides COOH puisque ces radicaux se séparent facilement en COO- (anion) et $H^+$ (cation), et convient donc pour capter des acides organiques.

Les résines échangeuses d'anions utilisées peuvent être par exemple les résines Dowex®1 fournie par Dow Chemical, USA et Amberlite® IRA-402 fournie par Rohm et Haas Co., USA

Selon la présente invention, les probiotiques sont incorporés en différenciation retardée, c'est-à-dire en fin de ligne de production et juste avant ou pendant l'étape de conditionnement.

De plus, l'étape b) et l'étape c) selon la présente invention peuvent être réalisées simultanément. Dans ce cas de figure, le procédé selon la présente invention possède seulement deux étapes pour éviter les altérations du produit alimentaire final lors de sa conservation, sans prolifération de microorganismes.

Dans un mode préférentiel, une étape d'ajout d'acide lactique sera effectué entre ou simultanément à l'étape b) et l'étape c) dudit procédé. La quantité d'acide lactique à ajouter sera déterminée facilement par l'homme du métier en fonction de la souche de bactérie qu'il souhaite mettre en oeuvre.

**Légende des figures :**

**Figure 1 :** Schéma métabolique d'assimilation du citrate et du malate, et de la production d'acétate chez les bactéries lactiques.

**Exemples**

(La souche DSM 9843 et les produits alimentaires comprenant la souche DSM 9843 ne font pas partie de l'invention.)

**EXEMPLE 1 :** Formation de gaz par les souches *L. plantarum* DSM 9843 et *L. plantarum* I-2845 (déposée à la CNCM le 4/04/02) en fonction du jus de fruits inoculé.

1 Matériel et Méthodes :

*I.1. Préparation des suspensions bactériennes et inoculation des jus de fruits*

Une première préculture de 2 mL avec les souches DSM 9843 et I-2845 est réalisée. Cette préculture sert à ensemencer à 1% 100 mL de MRS neutre (soit $10^8$-$10^9$ ufc/mL). A partir de cette deuxième préculture 3x 1000 mL en MRS neutre (soit $10^8$-$10^9$ ufc/mL) sont encemencés.

Pour chaque souche les centrifugations (Beckman JA-25, rotor JA-10) sont réalisées avec les pots de 500 mL de la façon suivante :

- remplissage de 6 pots avec 330 mL de culture
- centrifugation de 10 min, 12 000 g, 20°C
- élimination du surnageant et addition de 165 mL de culture
- centrifugation de 10 min, 12 000 g, 20°C
- élimination du surnageant

Chaque culot obtenu est ensuite repris séparément dans le jus de fruit à tester et la suspension obtenue est remise dans la brique de jus de fruit qui est soigneusement refermée ensuite.

*I.2. Dosages des acides organiques.*

La technique retenue consiste à séparer les acides organiques par chromatographie d'échanges d'anions haute performance (HPAEC). La détection des acides organiques est réalisée par conductimétrie suppressive (SCD).

Le système chromatographique utilisé est de marque DIONEX (type DX600) comprenant un système de détection par conductimétrie suppressive. La cellule de conductimétrie thermostatée (type DS3) est couplée à un système d'auto-suppression externe ASRS-ULTRA (4mm). Ce suppresseur électrolytique a été utilisé avec un mode de recirculation d'eau Milli-Q à contre-courant, à un débit de 4 mL/min (pression de 15 psi environ).

Une colonne d'échange d'anions de type AS11-HC (4mm) est associée à une colonne de garde de type AG11-HC. Le débit d'élution est de 1,5 mL/min.

II Résultats :

*II.1. Numérations bactériennes*

Des numérations bactériennes sont réalisées lors de la conservation des produits afm d'évaluer la survie de *L. plantarum* dans les matrices jus de fruits.

**Tableau 4 :** Numérations bactériennes de *L. plantarum* lors de la conservation à 10°C des matrices jus de fruits.

| Souche | Temps (j) | Orange | Pomme | Raisin |
|---|---|---|---|---|
| DSM 9843 | J0 | $1{,}8.10^9$ ufc/mL | $1{,}7.10^9$ ufc/mL | $9{,}5.10^8$ ufc/mL |
| | J5 | $5{,}0.10^9$ ufc/mL | $5{,}8.10^8$ ufc/mL | $4{,}1.10^9$ ufc/mL |
| I-2845 | J0 | $1{,}1.10^9$ ufc/mL | $9{,}8.10^8$ ufc/mL | $6{,}0.10^8$ ufc/mL |
| | J5 | $4{,}5.10^9$ ufc/mL | $1{,}6.10^9$ ufc/mL | $3{,}9.10^9$ ufc/mL |

*II.2. Mise en évidence de la consommation d'acides organiques lors du stockage.*

Les dosages des acides organiques ont été réalisés à 0 et 5 jours au même moment que les numérations et les résultats sont synthétisés dans le tableau 4.

**Tableau 5 :** Métabolites produits et acides organiques consommés lors de la conservation à 10°C de jus de fruit contenant *L. plantarum.*

| | | Lots | Gonflement de la bouteille | pH | Lactate produit | Acétate produit | Malate Consommé | Citrate consommé |
|---|---|---|---|---|---|---|---|---|
| | | | | | mmol | mmol | mmol | mmol |
| **Jus de pomme** | **Témoin** | **J0** | - | 3,43 | 0,00 | 0,00 | 0,00 | 0,00 |
| | **+ DSM 9843** | **J5** | ++ | 3,38 | 27,93 | 4,44 | 6,72 | 0,21 |
| | **+ I-2845** | **J5** | ++ | 3,39 | 40,86 | 4,38 | 21,20 | 0,19 |
| **Jus d'orange** | **Témoin** | **J0** | - | 3,34 | 0,00 | 0,00 | 0,00 | 0,00 |
| | **+ DSM 9843** | **J5** | +++ | 3,26 | 53,79 | 25,78 | 11,99 | 4,91 |
| | **+ I-2845** | **J5** | ++ | 3,27 | 48,90 | 15,60 | 13,26 | 1,42 |
| **Jus de raisin** | **Témoin** | **J0** | - | 3,22 | 0,00 | 0,00 | 0,00 | 0,00 |
| | **+ DSM 9843** | **J5** | ++ | 3,23 | 40,79 | 10,38 | 23,50 | 2,09 |
| | **+ I-2845** | **J5** | +++ | 3,24 | 44,59 | 8,16 | 33,87 | 1,43 |

D'après les résultats présentés dans le tableau 5, il apparaît clairement que l'acide malique est le substrat le plus consommé par *L. plantarum* quelle que soit la souche considérée. Cette consommation s'accompagne non seulement de productions de lactate et d'actétate et donc d'une baisse sensible du pH (notamment dans les jus d'orange et de pomme) mais aussi d'une production de gaz ayant un effet macroscopique sur l'emballage.

Selon les voies métaboliques présentées sur la figure 1, l'absence de détection de formiate produit (pas d'action de la pyruvate formiate lyase), la très faible teneur en pentoses des jus de fruits traités, le bilan de production de $CO_2$ (exprimé en moles) suivant peut être proposé :

$$CO_2 \text{ total} = \text{malate consommé} + \text{citrate consommé} + (\text{acétate total produit} - \text{acétate issu du citrate})$$

Soit, en remplaçant l'acétate produit à partir du citrate par la quantité de citrate consommé :

$$CO_2 \text{ total} = \text{malate consommé} + \text{acétate total produit}$$

Conclusion :

L'acide malique et, dans une moindre mesure, l'acide citrique contribuent donc fortement à la production de gaz lors de la conservation à 10°C de jus de fruits contenant une dose élevée (> $1.10^9$ ufc/mL) de bactérie *L. plantarum* DSM 9843 ou I-2845.

**EXEMPLE 2 :** dilution des jus d'oranges pour définir les taux limites en acides organiques compatibles avec le *L. plantarum* en fonction du pourcentage de jus dans la formule.

Nous avons pratiqué des dilutions à 5, 10, 20, 30% des jus d'oranges, ces dilutions correspondant à des taux de déacidifications de 95, 90, 80 et 70%.

**Tableau 6**

| % de jus dans le produit | % de déacidification | pH | Observation de gonflement ? |
|---|---|---|---|
| 30% | 70% | pH naturel | Gonflement à J+3 |
| 30% | 70% | 3,7 | Gonflement à J+5 |
| 20% | 80% | pH naturel | Léger gonflement à J+14 |
| 20% | 80% | 3,7 | Très faible gonflement |
| 10% | 90% | pH naturel | Non |
| 10% | 90% | 3,7 | Non |
| 5% | 95% | pH naturel | Non |
| 5% | 95% | 3,7 | Non |

Pour qu'une boisson à base de jus d'orange contenant *L. plantarum* soit stable plus de 30 jours après sa fabrication, nous avons déterminé qu'il fallait qu'elle ait les caractéristiques suivantes :

**Tableau 7 :**

| % de jus d'orange dans la boisson | Acidité du jus d'orange | Ratio Brix / acidité |
|---|---|---|
| 100 | 0,4-0,6 | 100-150 |
| 75 | 1,3-1,45 | 41-46 |
| 50 | 2,2-2,3 | 27-30 |
| Remarques : - le « rapport Brix/acide » désigne le rapport de la valeur Brix du jus au nombre de grammes d'acide citrique anhydre par 100 grammes de jus ; (*Brix/acid ratio*).<br>- la « valeur Brix » désigne la teneur en sucre déterminée par réfractométrie, à laquelle la correction pour l'acidité est ajoutée, selon la méthode de *l'Association of Official Analytical Chemist* des Etats-Unis, intitulée *Solids (Soluble) in Fruit Products,* publiée dans *Official Methods of Analysis of the Association of Official Analycical Chemist* 14e édition, 1984. *(Brix content)* DORS/88-8, art.2 ; DORS/95-548, art.2 ; DORS/2000-184, art.27 ; DORS/2003-6, art.65 (F). | | |

**EXEMPLE 3** : Analyse sensorielle de différents produits :

Suite aux problèmes techniques de mise en oeuvre de la souche DSM 9843 dans des milieux végétaux (production de $CO_2$ via la métabolisation d'acide malique et ou citrique conduisant au gonflement des briques UHT ; production de faux-goûts via la présence d'acides organiques et la métabolisation d'acides phénoliques), les solutions techniques suivantes ont été testées :

1) Déacidification des jus sur résine échangeuse d'ions,
2) Utilisation d'eau osmosée (pour évaluer l'impact des minéraux sur les faux-goûts),
3) Utilisation de différents types d'acides pour l'acidification : acide lactique, acide citrique ou acide ortho-phosphorique

A partir de toutes ces hypothèses, 7 essais ont été réalisés :

**Tableau 8 :**

| Essais | Type de jus d'orange (24%) | Présence de lait (16%) | Type d'acide pour pH 3,8 |
|---|---|---|---|
| SLC | Jus standard (S) | Oui (L) | Acide citrique (C) |
| DLC | Jus déacidifié (D) | Oui | Acide citrique |
| SLL | Jus standard | Oui | Acide lactique (L) |
| DLL | Jus déacidifié | Oui | Acide lactique |
| DLL osmosé | Jus déacidifié | Oui | Acide lactique |
| DL | Jus déacidifié | Non | Acide lactique |
| LL | Pas de jus | Oui | Acide lactique |

**Résultats :**

**Tableau 9 :**

| MIX | | Type d'acide | Essai | Arômes à J 0 | Arôme à J30 | BILAN |
|---|---|---|---|---|---|---|
| Jus standard | + lait | + acide citrique | SLC | Notes « jus » +++ Faux-goûts - | Notes « jus »+ Faux-goûts +++ (foin, terre, étable) | ↓ des notes « jus » ↑↑↑ des faux-goûts |
| | + lait | + acide lactique | SLL | Notes « jus »+++ faux-goûts- | Notes « jus » + Faux-goûts +++ (foin, terre, étable) | ↓ des notes « jus » ↑↑↑ des faux-goûts |
| Jus déacidifié | + lait | + acide citrique | DLC | Notes « jus » +++ Faux-goûts- | Notes « jus »-Faux-goûts +++ (vinaigre) | ↓ des notes « jus » ↑↑↑ des faux-goûts |
| | + lait | + acide lactique | DLL | Notes « jus » - Faux-goûts -- | Notes «jus »-Faux-goûts + (rance) | Pas de notes « jus » ↑ des faux-goûts |
| | + lait | + acide lactique | DLL Osmo | Notes « jus »- Faux-goûts -- | Notes « jus »-Faux-goûts - (un peu fruité/floral) | Pas de notes « jus » Pas de faux-goûts |
| | + lait | + acide lactique | DL | Notes « jus » +++ faux-goûts --- | Notes « jus »-Faux-goûts - (un peu jus/fruité) | ↓ des notes « jus » Pas de faux-goûts |
| Lait | | + acide lactique | LL | Notes « jus »- Faux-goûts -- | Notes « jus »-Faux-goûts -(très fruité/floral) | Pas de notes « jus » Pas de faux-goûts |
| Remarque : les arômes son évalués de - (intensité très faible de cet arôme) à +++ (intensité très forte de cet arôme). Une flèche vers le bas dans le tableau (↓) indique une diminution, une flèche vers le haut (↑) une augmentation et trois flèche vers le haut (↑↑↑) une augmentation importante des notes aromatiques positives (notes « jus ») et/ou négatives (faux goûts) pour le jus. | | | | | | |

D'une manière générale, même si l'acide citrique renforce les notes «jus d'orange » dans les produits à J0, à J30 tous les produits ont perdu ces caractéristiques organoleptiques et sont relativement neutres au niveau des notes

fruitées/orange.

Au niveau métabolique, l'acide citrique est bien un précurseur de faux-goûts, car cet acide est métabolisé par *Lactobacillus plantarum* pour former de l'acide acétique (dans le cas de jus déacidifié) ou de l'éthyl phénol (dans le cas de jus standard). Par conséquent, l'ajout d'acide citrique doit être le plus faible possible pour éviter la formation de ces molécules, tout en ayant un effet positif sur les notes « jus ».

Au niveau du type de jus, la déacidification des jus est un procédé permettant d'éviter la formation des faux-goûts (excepté en présence d'acide citrique) et seules des notes rances persistent, principalement dues à la présence de lait. Par conséquent, un ajustement du ratio jus déacidifié/lait doit être réalisé.

Au final, les meilleurs résultats en terme d'absence de faux-goûts (foin, étable, terre, vinaigre, rance) et de présence de notes aromatiques caractéristiques des jus d'orange, sont obtenus avec du jus déacidifié mélangé avec de l'eau osmosée et acidifiée à l'acide lactique.

**EXEMPLE 4** : formule du type jus de fruit + lait

<u>**Tableau 10 :**</u>

| **Produit Jus de fruit Orange + Lait à pH cible 3,8** | |
|---|---|
| Eau | Environ 70% |
| Sucre | Environ 7,5% |
| Jus d'orange concentré déacidifié | Environ 4,5% |
| Arôme Orange | 0,0054% |
| Pectine | 0,56% |
| Beta Carotène | 0,09% |
| Lait | Environ 16,5% |
| Acide lactique | pH final 3,8 |
| *Lactobacillus plantarum* DSM 9843 | Ensemencé 0,1% |

**EXEMPLE 5 :** formules orange 50% et 75% de jus.

<u>**Tableau 11 :**</u>

| **Jus de fruit Orange 50% à pH cible 3,6** | |
|---|---|
| Eau | 80,4 |
| Sucre | 3,5 |
| Jus d'orange concentré déacidifié | 11 |
| Pulpe | 3,7 |
| Pectine | 0,2 |
| Colorant | 0,02 |
| Acide lactique | 0,4 |
| Acide ascorbique | 0,03 |
| Souche de *L. plantarum* DSM 9843 | Ensemencé à 0,1% |

**Tableau 12 :**

| Jus de fruit Orange 75% à pH cible 3,6 | |
|---|---|
| Eau | 80,2 |
| Sucre | 1,3 |
| Jus d'orange concentré désacidifié | 13,4 |
| Pulpe | 3,9 |
| Pectine | 0,2 |
| Colorant | 0,02 |
| Acide lactique | 0,13 |
| Acide ascorbique | 0,03 |
| Souche de *L. plantarum* DSM 9843 | Ensemencé à 0,1% |

## Revendications

1. Procédé de préparation d'un produit alimentaire conditionné à base de fruits, **caractérisé en ce qu'**il comprend les étapes suivantes :

    a) Appauvrissement en acides organiques d'une matrice à base de fruits,
    b) Ajout de probiotiques à la matrice obtenue après l'étape a),
    c) Conditionnement du produit obtenu après l'étape b),

    dans lequel le produit alimentaire conditionné à base de fruits comprend entre 50% et 99.99% de jus ou de purée de fruits et des probiotiques *Lactobacillus plantarum* vivants déposée le 4/04/02 sous le numéro CNCM 1-2845 à la Collection Nationale des Cultures de Microorganismes,
    le produit alimentaire a une teneur en acides organiques diminuée de 10 à 100%, préférentiellement de 30 à 70 % encore plus préférentiellement de 60% par rapport à la teneur initiale de la matrice de fruits en acides organiques et la production de faux-goûts dans le produit alimentaire est réduite ou éliminée par rapport à la matrice de fruits initiale.

2. Procédé selon la revendication 1 **caractérisé en ce que** l'étape a) d'appauvrissement en acides organiques de la matrice à base de fruits est réalisée par sélection d'une matrice de fruits à faible acidité naturelle, ladite matrice étant une matrice de fruits à partir de laquelle on obtient un jus qui a une acidité comprise entre la valeur basse indiquée sur le « Code of Practice » de l'AIJN et -50% de cette valeur.

3. Procédé selon la revendication 2 **caractérisé en ce que** la sélection d'une matrice de fruits à faible acidité naturelle est réalisée par sélection variétale des fruits et/ou contrôle de la maturité des fruits.

4. Procédé selon la revendication 1 **caractérisé en ce que** l'étape a) d'appauvrissement en acides organiques de la matrice à base de fruits est réalisée par déacidification de la matrice de fruits.

5. Procédé selon la revendication 4 **caractérisé en ce que** la diminution de l'acidité titrable de la matrice de fruits est réalisée par électrodialyse de la matrice de fruits, précipitation des acides organiques de la matrice de fruits par des sels de calcium, fermentation malolactique, assimilation sélective de l'acide citrique, et/ou passage de la matrice de fruits sur une résine échangeuse d'anions.

6. Procédé selon l'une quelconque des revendications 1 à 5 **caractérisé en ce que** l'étape b) et l'étape c) sont réalisées simultanément.

7. Procédé selon l'une quelconque des revendications 1 à 6 **caractérisé en ce que** la matrice à base de fruits est un jus de fruits, un jus de fruits reconstitué à base de concentré, ou une purée de fruits.

8. Procédé selon l'une quelconque des revendications 1 à 7, **caractérisé en ce qu'**une étape d'ajout d'acide lactique

sera effectuée entre ou simultanément à l'étape b) et/ou l'étape c) dudit procédé.

9. Souche de *Lactobacillus plantarum* déposée le 4/04/02 sous le numéro CNCM 1-2845 à la Collection Nationale des Cultures de Microorganismes.

## Patentansprüche

1. Verfahren für die Herstellung eines verpackten Lebensmittelprodukts auf Fruchtbasis, **dadurch gekennzeichnet, dass** es die folgenden Schritte umfasst:

   a) Abreichern der organischen Säuren in einer Matrix auf Fruchtbasis,
   b) Hinzufügen von Probiotika zu der Matrix nach Schritt a),
   c) Verpacken des nach Schritt b) erhaltenen Produkts,

   wobei das verpackte Lebensmittelprodukt auf Fruchtbasis zwischen 50 % und 99,99 % Fruchtsaft oder püree und lebende Probiotika *Lactobacillus plantarum,* hinterlegt am 4.04.02 unter der Nummer CNCM I-2845 bei der Collection Nationale des Cultures de Microorganismes, umfasst,
   wobei das Lebensmittelprodukts einen von 10 bis 100%, vorzugsweise von 30 bis 70 %, noch vorzugsweiser von 60 %, verringerten Gehalt an organischen Säuren in Bezug zum Ausgangsgehalt der Fruchtmatrix an organischen Säuren hat und die Ausbildung von Nebengeschmäckern in dem Lebensmittelprodukt in Bezug zur Ausgangs-Fruchtmatrix reduziert oder beseitigt ist.

2. Verfahren nach Anspruch 1, **dadurch gekennzeichnet, dass** der Schritt a) der Abreicherung der organischen Säuren in der Matrix auf Fruchtbasis durch Auswahl einer Fruchtmatrix mit schwachem natürlichem Säuregehalt durchgeführt wird, wobei die Matrix eine Fruchtmatrix ist, aus der man einen Saft erhält, der einen Säuregehalt zwischen einem niedrigen Wert, angegeben im "Code of Practice" der AIJN, und -50 % dieses Werts inklusive hat.

3. Verfahren nach Anspruch 2, **dadurch gekennzeichnet, dass** die Auswahl einer Fruchtmatrix mit niedrigem natürlichem Säuregehalt durch Sortenauswahl von Früchten und/oder Überwachung der Reife der Früchte durchgeführt wird.

4. Verfahren nach Anspruch 1, **dadurch gekennzeichnet, dass** der Schritt a) der Abreicherung der organischen Säuren in der Matrix auf Fruchtbasis durch Entsäurerung der Fruchtmatrix durchgeführt wird.

5. Verfahren nach Anspruch 4, **dadurch gekennzeichnet, dass** die Verringerung des titrierbaren Säuregehalts der Fruchtmatrix durch Elektrodialyse der Fruchtmatrix, Ausfällen der organischen Säuren der Fruchtmatrix mit Hilfe von Calciumsalzen, malolaktische Fermentation, selektive Assimilation der Citronensäure und/oder Durchgang der Fruchtmatrix durch ein Anionentauscherharz durchgeführt wird.

6. Verfahren nach einem der Ansprüche 1 bis 5, **dadurch gekennzeichnet, dass** der Schritt b) und der Schritt c) gleichzeitig durchgeführt werden.

7. Verfahren nach einem der Ansprüche 1 bis 6, **dadurch gekennzeichnet, dass** die Matrix auf Fruchtbasis ein Fruchtsaft, ein auf Basis von Konzentrat wiederhergestellter Fruchtsaft oder ein Fruchtpüree ist.

8. Verfahren nach einem der Ansprüche 1 bis 7, **dadurch gekennzeichnet, dass** ein Schritt des Hinzufügend von Milchsäure zwischen oder gleichzeitig mit Schritt b) und/oder Schritt c) des Verfahrens durchgeführt wird.

9. Stamm von *Lactobacillus plantarum,* hinterlegt am 4.04.02 unter der Nummer CNCM I-2845 bei der Collection Nationale des Cultures de Microorganismes.

## Claims

1. A method of preparing a packaged fruit-based food product, **characterized in that** it comprises the following steps:

   a) Depletion of organic acids from a fruit-based matrix,

b) Addition of probiotics to the matrix obtained after step a),
c) Packaging of the product obtained after step b),

wherein the packaged fruit-based food product contains between 50 and 99.99% fruit juice or fruit puree and live *Lactobacillus plantarum probiotics* deposited at the Collection Nationale des Cultures de Microorganismes on 04/04/02, under the number CNCM I-2845,
wherein the food product has an organic acid content reduced by 10 to 100%, preferably by 30 to 70%, and more preferably yet by 60%, in relation to the initial organic acid content of the fruit matrix and wherein the production of off-tastes in the food product is reduced or eliminated in relation to the initial fruit matrix.

**2.** The method according to claim 1, **characterized in that** step a) for depleting organic acids from a fruit-based matrix is carried out by selecting a fruit matrix having low natural acidity, wherein said matrix is a fruit matrix from which a juice is obtained, the acidity of which is between the low value indicated in the AIJN "Code of Practice" and -50% of this value.

**3.** The method according to claim 2, **characterized in that** the selection of a fruit matrix having a low natural acidity is carried out via varietal selection of the fruits and/or by controlling the ripening of the fruits.

**4.** The method according to claim 1, **characterized in that** the step a) for depleting organic acids from a fruit-based matrix is carried out via de-acidification of the fruit matrix.

**5.** The method according to claim 4, **characterized in that** the reduction in titratable acidity of the fruit matrix is carried out via electrodialysis of the fruit matrix, precipitation of the organic acids from the fruit matrix with calcium salts, malolactic fermentation, selective assimilation of the citric acid and/or passing the fruit matrix over an anion exchange resin.

**6.** The method according to any one of claims 1 to 5, **characterized in that** step b) and step c) are carried out simultaneously.

**7.** The method according to any one of claims 1 to 6, **characterized in that** the fruit-based matrix is a fruit juice, a concentrate-based reconstituted fruit juice, or a fruit puree.

**8.** The method according to any one of claims 1 to 7, **characterized in that** step for adding lactic acid is carried out between step b) and step c) of said method, or simultaneously therewith.

**9.** A strain of Lactobacillus plantarum deposited at the Collection Nationale des Cultures de Microorganismes on 04/04/02, under the number CNCM I-2845.

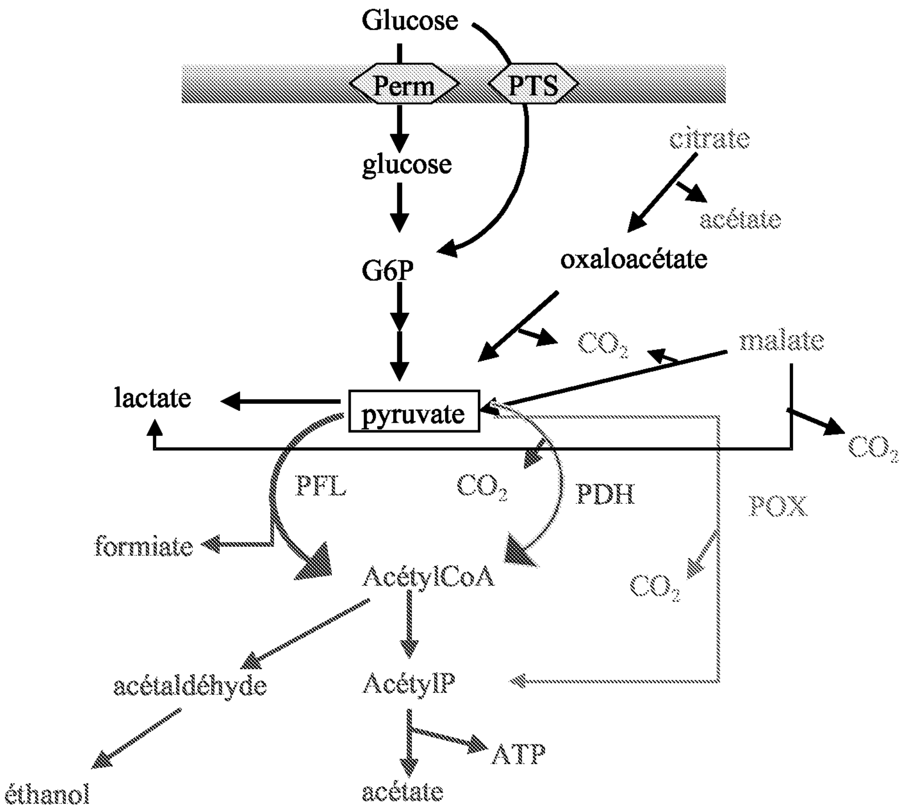
Glucose
Perm
PTS
glucose
G6P
citrate
acétate
oxaloacétate
CO2
malate
lactate
pyruvate
CO2
PFL
CO2
PDH
POX
formiate
AcétylCoA
CO2
acétaldéhyde
AcétylP
ATP
éthanol
acétate

Figure 1

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 0070972 A
- EP 0113055 A
- EP 0166238 A


### Littérature non-brevet citée dans la description


- **VAN BEEK, S ; PRIEST FG.** Decarboxylation of subsituted cinnamic acids by lactic acid bacteria isolated during malt whisky fermentation. *Applied and Environmental Microbiology,* 2000, vol. 66 (12), 5322-8
- **HEGAZI F.Z. ; ABO-ELNAGA I.G.** Dégradation of organic acids by dairy lactic acid bacteria. *Mikrobiologie der Landwirtschaft der Technologie und des Umweltschutzes,* 1980, vol. 135 (3), 212
- **HARTWIG P. ; MCDANIEL M.R.** Flavor characterisitics of lactic, malic, citric and acetic acids at various pH levels. *Journal of Food Science,* 1995, vol. 60 (2), 384-388